(19) Europäisches Patentamt
European Patent Office
Office européen des brevets

(11) Veröffentlichungsnummer : **0 069 330**
**B1**

(12) **EUROPÄISCHE PATENTSCHRIFT**

(45) Veröffentlichungstag der Patentschrift : **09.01.85**

(51) Int. Cl.⁴ : **C 07 D213/30**, A 01 N 43/40

(21) Anmeldenummer : **82105805.4**

(22) Anmeldetag : **30.06.82**

(54) **Pyridincarbinole, Verfahren zu ihrer Herstellung und diese enthaltende Fungizide.**

(30) Priorität : **08.07.81 DE 3126819**

(43) Veröffentlichungstag der Anmeldung : **12.01.83 Patentblatt 83/02**

(45) Bekanntmachung des Hinweises auf die Patenterteilung : **09.01.85 Patentblatt 85/02**

(84) Benannte Vertragsstaaten :
**AT BE CH DE FR IT LI NL SE**

(56) Entgegenhaltungen :
**EP-A- 0 001 399**
**CH-A- 498 568**
**US-A- 3 655 359**
**US-A- 4 039 675**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Buschmann, Ernst, Dr.**
**Georg-Ludwig-Krebs-Strasse 10**
**D-6700 Ludwigshafen (DE)**
Erfinder : **Ammermann, Eberhard, Dr.**
**Sachsenstrasse 3**
**D-6700 Ludwigshafen (DE)**
Erfinder : **Pommer, Ernst-Heinrich, Dr.**
**Berliner Platz 7**
**D-6703 Limburgerhof (DE)**

**EP 0 069 330 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Pyridincarbinole und Salze dieser Verbindungen sowie fungizide Mittel, die diese Verbindungen enthalten und Verfahren zu ihrer Herstellung.

Fungizide Pyridinderivate beispielsweise das 5-Butyl-5-(4-tert.-butylbenzyl)-3-pyridyl-iminodithiocarbonat werden in der japanischen Patentanmeldung 72/43334 beschrieben.

Es ist ferner bekannt, dialkylsubstituierte 3-Pyridinmethanole (US-A-4 039 675) oder Pyridinbasenverbindungen (CH-A-498 568) als Fungizide zu verwenden. Es sind ferner substituierte 3-Pyridinmethane bekannt (US-A-3 655 359). Durch die bekannten Verbindungen werden die neuen Verbindungen nicht nahegelegt, weil die neuen Verbindungen eine andere chemische Struktur oder eine überraschend bessere fungizide Wirkung haben.

Es wurde nun gefunden, daß Pyridincarbinole der Formel

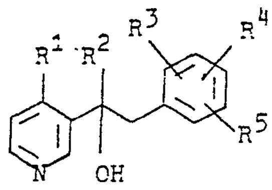

(I)

in der $R^1$ Wasserstoff oder einen $C_1$-$C_6$-Alkylrest, $R^2$ einen $C_1$-$C_6$-Alkylrest, $R^3$ und $R^4$ Chlor und $R^5$ Wasserstoff bedeuten und die pflanzenphysiologisch verträglichen Säureadditionssalze dieser Verbindungen eine gute fungizide Wirkung haben, die der Wirkung der bekannten Pyridinderivate überlegen ist.

$R^1$ bedeutet beispielsweise Wasserstoff, Methyl, Ethyl, Propyl, Butyl, Isobutyl, Pentyl, Hexyl.

$R^2$ steht zum Beispiel für Methyl, Ethyl, Propyl, Butyl, Isobutyl, Pentyl, 3,3-Dimethyl-butyl, Hexyl.

Pflanzenphysiologisch verträgliche Salze der Verbindungen sind z. B. Hydrochloride, Hydrobromide, Sulfate, Phosphate, Oxalate, Acetate, Formiate, oder Additionsverbindungen mit Säuren von Tensiden, z. B. Dodecylbenzolsulfonsäure.

Die Verbindungen enthalten mindestens ein asymmetrisches C-Atom. Sie kommen daher in Form ihrer optischen Isomeren vor. Die Erfindung umfaßt sowohl die reinen optischen Isomeren als auch ihre Mischungen.

Das Verfahren zur Herstellung der neuen Verbindungen ist dadurch gekennzeichnet, daß man ein Acylpyridin der Formel

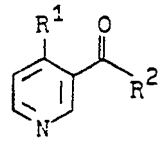

(II)

in der $R^1$ und $R^2$ die oben genannten Bedeutungen haben, in Gegenwart eines Lösungsmittels umsetzt mit metallorganischen Verbindungen der Formel

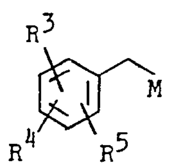

(III)

in der M für Li, Na, MgBr und MgCl steht und $R^3$, $R^4$ und $R^5$ die oben genannten Bedeutungen haben.

Geeignete Lösungsmittel sind beispielsweise: Diethylether, Tetrahydrofuran, Dimethoxyethan, Toluol.

Acylpyridine der Formel II sind teilweise bekannt aus R.L. Frank und C. Weatherbee J. Am. Chem. Soc. *70,* 3482 (1948). Die dort für $R^1$ = H, Propyl, Butyl und $R^2$ = Propyl, Butyl, gegebenen Versuchsvorschriften können ohne Schwierigkeit auch auf andere Alkylreste übertragen werden.

Ein weiterer Syntheseweg für 3-Acylpyridine der Formel II wird im folgenden Schema aufgezeigt:

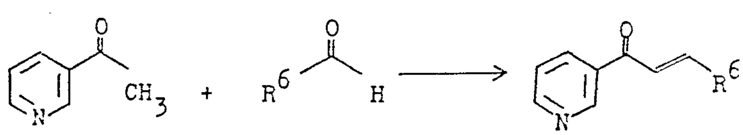

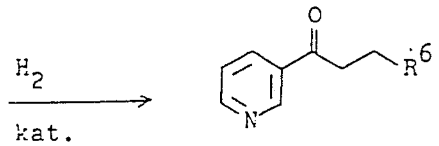


$R^6$ = Alkyl

Die folgenden Vorschriften und das Beispiel erläutern die Herstellung der Vorprodukte und der neuen Verbindungen.

Vorschrift I

3,3-Dimethylbutyl-pyridin-3-yl-keton

In eine Lösung von 200 g β-tert.-Butylvinyl-pyridin-3-ylketon in 400 ml Wasser wird nach Zugabe von 20 g Hydrierkatalysator (5 % Pr, 0,5 % Pd auf $Al_2O_3$) und $N_2$-Spülung Wasserstoff eingepreßt. Man rührt 8 Stunden bei 130 °C und 20 Bar. Die Wasserstoffeinleitung wird dabei bis zu Druckkonstanz fortgesetzt. Nach Abkühlen und Abfiltrieren des Katalysators wird das Filtrat eingeengt und destilliert. Man erhält 75 g 3,3-Dimethylbutyl-pyridin-3-yl-keton, Sdp. 110-116 °C, 0,6 mbar.

Beispiel 1

3,3-Dimethylbutyl-2,4-dichlorbenzyl-pyridin-3-yl-carbinol

Zu 8,2 Mg in 100 ml Diethylether wurden zugetropft eine Lösung von 66,2 g 2,4-Dichlorbenzylchlorid in 150 ml Ether. Nach Zugabe von 20 % der Dichlorbenzylchloridmenge wurde die Reaktion durch Zugabe einiger Tropfen Brom zum Anspringen gebracht. Nach Beendigung des Zutropfens der 2,4-Dichlorbenzylchlorid-Lösung wurde 1/2 Stunde zur Rückfluß erwärmt. Anschließend tropfte man zu eine Lösung von 55 g 3,3-Dimethylbutyl-pyridin-3-yl-keton in 100 ml Ether. Nach zwei weiteren Stunden Rückfluß wurde das Reaktionsgemisch unter Eiskühlung durch Zutropfen von gesättigter wäßriger $NH_4Cl$-Lösung (bis pH = 8) hydrolysiert. Man rührte 15 Stunden bei Raumtemperatur (20 °C), extrahierte mit Diethyl-Ether, wusch die etherische Phase mit Wasser, trocknete über $Na_2SO_4$, engte ein und destillierte. Man erhielt 52 g 3,3-Dimethylbutyl-2,4-dichlorbenzyl-pyridin-3-yl-carbinol, Sdp. 196-202 °C, 0,5 mbar (Wirkstoff Nr. 36).

Entsprechend wurden diejenigen in der folgenden Tabelle angegebenen Verbindungen hergestellt, deren Schmelzpunkte (Fp) oder Siedepunkte angegeben sind. Ihre Struktur wurd durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden ; es kann erwartet werden, daß sie aufgrund ihrer ähnlichen Konstitution ähnliche Wirkungen wie die näher untersuchten Verbindungen haben.

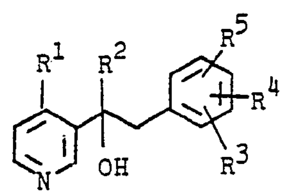
. HX

| Nr. | $R^1$ | $R^2$ | $R^3$, $R^4$, $R^5$ | HX | Sdp. °C/mbar Fp. |
|---|---|---|---|---|---|
| 4 | $CH_3$ | $CH_3$ | 2,4-$Cl_2$ | - | |
| 5 | H | $CH_3$ | 2,4-$Cl_2$ | - | 196-202/0,3 |
| 6 | H | $CH_3$ | 2,4-$Cl_2$ | HCl | 232 |
| 7 | H | $CH_3$ | 3,4-$Cl_2$ | - | 206/0,3 |
| 11 | H | n-$C_3H_7$ | 2,4-$Cl_2$ | - | 211/0,3 |
| 12 | H | n-$C_3H_7$ | 2,4-$Cl_2$ | HCl | 141 |
| 13 | n-$C_3H_7$ | n-$C_3H_7$ | 2,4-$Cl_2$ | - | 218-222/0,2 |
| 14 | H | n-$C_3H_7$ | 2,4-$Cl_2$ | $HNO_3$ | |
| 16 | H | iso-$C_3H_7$ | 2,4-$Cl_2$ | - | |

(Fortsetzung)

| Nr. | $R^1$ | $R^2$ | $R^3$, $R^4$, $R^5$ | HX | Sdp. °C/mbar Fp. |
|---|---|---|---|---|---|
| 19 | H | n-Butyl | 2,4-$Cl_2$ | - | 198-200/0,1 |
| 20 | H | n-Butyl | 2,4-$Cl_2$ | HCl | 130 |
| 22 | H | n-Butyl | 2,4-$Cl_2$ | 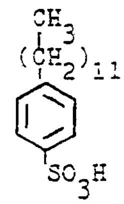 | Harz |
| 23 | H | n-Butyl | 2,4-$Cl_2$ | Oxalsäure | |
| 24 | n-Butyl | n-Butyl | 2,4-$Cl_2$ | | 235-238/ 0,1 |
| 25 | n-Butyl | n-Butyl | 2,4-$Cl_2$ | HCl | 159 |
| 30 | H | n-Pentyl | 2,4-$Cl_2$ | - | 195-206/0,3 |
| 31 | H | n-Pentyl | 2,4-$Cl_2$ | HCl | 120 |
| 32 | n-Pentyl | n-Pentyl | 2,4-$Cl_2$ | - | 223-228/0,2 |
| 33 | n-Pentyl | n-Pentyl | 2,4-$Cl_2$ | HCl | 149 |
| 34 | H | $(CH_2)_2CH(CH_3)_2$ | 2,4-$Cl_2$ | - | 196-202/0,3 |
| 35 | H | $(CH_2)_2CH(CH_3)_2$ | 2,4-$Cl_2$ | HCl | 133 |
| 36 | H | $(CH_2)_2$-t.-Butyl | 2,4-$Cl_2$ | - | 196-202/0,5 |
| 37 | H | $(CH_2)_2$-t.-Butyl | 2,4-$Cl_2$ | HCl | 177 |
| 47 | H | n-Propyl | 2,4-$Cl_2$ | 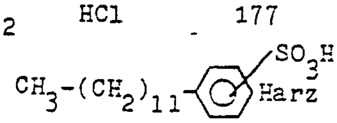 | Harz |
| 48 | H | n-Butyl | 2,4-$Cl_2$ | HCl | 180 |

Die neuen Verbindungen und ihre Salze zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Boden-fungizide eingesetzt werden.

Besonders interessant sind die fungiziden Verbindungen für die Bekämpfung einer Vielzahl von Pilzen an verschiedenen Kulturplanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, in Gemüsen wie Gurken, Bohnen oder Kürbisgewächsen sowie Reben im Weinbau;

Die neuen Verbindungen sind insbesondere geeignet zur Bekämpfung folgender Pflanzenkrankheiten :

Erysiphe graminis (echter Mehltau) in Getreide,
Erysiphe cichoracearum (echter Mehltau) an Kürbisgewächsen
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie Helminthosphoriumarten an Getreide,
Ustilago-Arten an Getreide und Zuckerrohr,
Rhynchosporium secale an Getreide und ganz besonders Venturia inaequalis (Apfelschorf) und außerdem
Botrytis cinerea an Reben, Paprika und Erdbeeren,
Monilia fructigena an Äpfeln,
Oidium heveae an Gummibäumen,
Oidium mangiferae an Mango,
Leivellula taurica an Bohnen, Erbsen und Tomaten,
Marssonina rosae an Rosen,
Cercospora musae an Bananen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser ; Trägerstoffe wie natürliche Gesteinsmehle (z. B. Kaoline, Tonerden, Talkum, Kreide) und synthetische Gesteinsmehle (z. B. hochdisperse Kieselsäure, Silikate) ; Emulgiermittel wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha.

Die Mittel bzw. die daraus hergestellten gebrauchsfertigen Zubereitungen wir Lösungen, Emulsionen, Suspensionen, Pulver, Stäube, Pasten, oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gewichtsteile der Verbindung 5 mit 10 Gewichsteilen N-Methyl-alpha-pyrrolidon und erhält eine Lösung, die zur Anwendung in Form kleinster Tropfen geeignet ist.

II. 20 Gewichtsteile der Verbindung 6 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsaure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält eine wäßrige Dispersion.

III. 20 Gewichtsteile der Verbindung 7 werden in einer Mischung gelöst, die aus 40 Gewichtsteilen Cyclohexanon, 30 Gewichtsteilen Isobutanol. 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung 11 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man ein wäßrige Dispersion.

V. 80 Gewichtsteile der Verbindung 12 werden den mit 3 Gewichtsteilen des Natriumsalzes der Diisobutyl-naphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammmermühle vermahlen. Durch feines Verteilen der Mischung in Wasser erhält man eine Spritzbrühe.

VI. 3 Gewichtsteile der Verbindung 13 werden mit 97 Gewichtsteilen feinteiligem Kaolin innig vermischt. Man erhält auf diese Weise ein Stäubemittel, das 3 Gew.-% des Wirkstoffs enthält.

VII. 20 Teile der Verbindung 20 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden mit denen die erfindungsgemäßen Verbindungen kominiert werden können, soll die Kombinationsmöglichkeiten erläutern, nicht aber einschränken.

Fungizide, die mit den erfindungsgemäßen Verbindungen kombiniert werden können, sind beispielsweise :

Schwefel,
Dithiocarbamate und deren Derivate, wie
Ferridimethyldithiocarbamat,
Zinkdimethyldithiocarbamat,

Manganethylenbisdithiocarbamat,
Mangan-Zink-ethylendiamin-bis-dithiocarbamat und
Zinkethylbisdithiocarbamat,
Tetramethylthiuramdisulfide,
Ammoniak-Komplex von Zink-(N,N-ethylen-bis-dithiocarbamat)
und N,N'-Polyethylen-bis-(thiocarbamoyl)-disulfid,
Zink-(N,N'-pyrolen-bis-dithiocarbamat),
Ammoniak-Komplex von Zink-(N,N'-propylen-bis-dithiocarbamat)
und N,N'-Polypropylen-bis-(thiocarbamoyl)-disulfid ;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Substanzen, wie
N-(1,1,2,2-Tetrachlorethylthio)-tetrahydrophthalimid,
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyi)-3-phenyl-1,2,4-triazol,

5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
N,N'-[1,4-Piperazindiyl-bis-(2,2,2-trichlor-ethylyliden)]-bis-formamid,
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und weitere Substanzen wie
Dodecylguanidinacetat,
3-(3-(3,5-dimethyl-2-oxycyclohexyl)-2-hydroxyethyl)-glutaramid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl(2)-alaninat,
DL-N-(2,6-Dimethyl-phenyl)-N-(2'-methoxyacetyl)-alaninmethylester,
5-Nitro-isophthalsäure-di-isopropylester,
1-(1',2',4'-Triazolyl-1')-1-(4'-chlorphenoxy)-3,3-dimethylbutan-2-on,
1-(1',2'-4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-propyl)-N-(2,4,6-trichlorphenoxyethyl)-N'-imidazolylharnstoff.

2,4,5-Trimethyl-furan-3-carbonsäureanilid
5-Methyl-5-vinyl-3-(3,5-dichlorphenyl)-2,4-dioxo-1,3-oxazolidin
5-Methoxymethyl-5-methyl-3-(3,5-dichlorphenyl)-2,4-dioxo-1,3-oxazolidin
N-[3-(p-tert.-Butylphenyl)-2-methyl-propyl]-cis-2,6-dimethylmorpholin.
1-(2,4-Dichlorphenyl)-4,4-dimethyl-2-(1,2,4)triazol-1-yl)-pentan-3-ol
α-(2-Chlorphenyl)-α-(4-fluorphenyl)-5-pyrimidin-methanol
α-(2-Chlorphenyl)-α-(4-chlorphenyl)-5-pyrimidin-methanol
β-[(1,1'-Biphenyl)-4-yl-oxy]-α-(1,1-dimethylethyl)-1H-1,2,4-triazol-1-ethanol
1-[2-(2,4-Dichlorphenyl)-4-ethyl-1,3-dioxolan-2-yl-methyl]-1H-1,2,4-triazol
1-[2-(2,4-Dichlorphenyl)-4-propyl-1,3-dioxolan-2-yl-methyl]-1H-1,2,4-triazol
1-[N-Propyl-N-2-(2,4,6-trichlorphenoxy)-ethyl]-carbamoylimidazol
2-Cyan-N-(ethylaminocarbonyl)-2-(methoxyimino)-acetamid
N-(1-Formylamido-2,2,2-trichlor-ethyl)-morpholin.

Für die folgenden Versuche wurde als bekanntes Vergleichsmittel die Verbindung 5-Butyl-5-(4-tert'-butylbenzyl)-3-pyridylimino-dithiocarbonat (A) verwendet.

Versuch 1

Wirksamkeit gegen Weizenmehltau

Blätter von in Töpfen gewachsenen Weizenkeimlingen der Sorte « Jubilar » werden mit wäßriger Spritzbrühe, die 80 % (Gew.-%) Wirkstoff und 20 % Emulgiermittel in der trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 7 Tagen wird das Ausmaß der Mehltauentwicklung ermittelt.

Das Ergebnis des Versuchs zeigt, daß beispielsweise die Verbindungen 5, 6, 11, 12, 19, 20, 30, 31, 34, 35 bei Anwendung als 0,025 %ige, 0,006 %ige und 0,001 5 %ige Spritzbrühen eine bessere fungizide Wirkung (beispielsweise 97 %) haben als der Wirkstoff A (beispielsweise 90 %).

Versuch 2

Wirksamkeit gegen Gurkenmehltau

Blätter von in Töpfen gewachsenen Gurkenkeimlingen der Sorte « Chinesische Schlange » werden im Zweiblattstadium mit einer Sporensuspension des gurkenmehltaus (Erysiphe cichoracearum) besprüht. Nach etwa 20 Stunden werden die Versuchspflanzen mit wäßriger spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22 °C und 70 bis 80 % relativer Luftfeuchtigkeit aufgestellt. Zur Beurteilung der Wirksamkeit der neuen Stoffe wird das Ausmaß der Pilzentwicklung nach 14 Tagen beurteilt.

Das Ergebnis des Versuches zeigt, daß beispielsweise die Wirkstoffe 5, 6, 7, 11, 12, 13, 19, 20, 24, 25, 30, 31, 32, 33, 34 und 35 bei Anwendung als 0,025 %ige Spritzbrühe eine gute fungizide Wirkung haben (beispielsweise 100 %).

Versuch 3

Wirksamkeit gegen Weizenbraunrost

Blätter von in Töpfen gewachsenen Weizensämlingen der Sorte « Jubilar » werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschlißend mit wäßrigen Spritzbrühen, die 80 % Wirkstoff enthalten, tropfnaß gespritzt. Nach dem Antrocknen des Spritzbelages werden die Versuchspflanzen im Gewächshaus bei Temperaturen zwischen 20 und 22 °C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß beispielsweise die Wirkstoffe 11, 19, 20, 30, 31 und 35 bei Anwendung als 0,025 %ige Spritzbrühe eine bessere fungizide Wirkung (beispielsweise 97 %) haben als die Verbindung A (beispielsweise 60 %).

Versuch 4

Wirksamkeit gegen Botrytis cinerea an Paprika

Paprikasämlinge der Sorte « Neusiedler Ideal Elite » werden, nachdem sich 4-5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22-24 °C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß die Wirkstoffe 6, 7, 11, 12, 13, 19, 24, 30, 31, 32, 34, 35 und 47 bei Anwendung als 0,025 %ige Spritzbrühe eine bessere fungizide Wirkung (beispielsweise 97 %) haben als die Verbindung A (beispielsweise 90 %).

Versuch 5

Wirksamkeit gegen Schorf

Junge Blätter von in Töpfen gewachsenen Apfelsämlingen der Sorte « Golden Delicious » werden mit wäßriger Spritzbrühe, die 80 % Wirkstoff und 20 % Emulgiermittel in der Trockensubstanz enthält, tropfnaß gespritzt. Nach dem Antrocknen des Spritzbelages werden die Versuchspflanzen mit einer Sporensuspension des Apfelschorfs (Venturia inaequalis) besprüht. Anschließend werden die inoculierten Pflanzen in einer Klimakammer bei 20 bis 22 °C und 95 % relativer Luftfeuchtigkeit für 10 Tage aufgestellt. Dann wird das Ausmaß der Pilzentwicklung auf den Blättern ermittelt.

Das Ergebnis des Versuches zeigt, daß Versuches zeigt, daß beispielsweise die Verbindungen 5, 7, 11, 12, 13, 19, 24, 30, 34 und 35 bei Anwendung als 0,007 5 %ige Spritzbrühe eine gute fungizide Wirkung haben (beispielsweise 97 %).

Versuch 6

Wirksamkeit gegen Rebenmehltau (Uncinula necator)

Topfreben der Sorte « Müller-Thurgau » mit 5-6 gut entwickelten Blättern werden mit wäßriger Spritzbrühe, die 80 % Wirkstoff und 20 % Emulgiermittel in der Trockensubstanz enthält, tropfnaß besprüht. Nach dem Antrocknen des Spritzbelages werden die Pflanzen mit einer wäßrigen Sporensuspension von Uncinula necator inoculiert und im Gewächshaus für Tage aufgestellt. Dann wird das Ausmaß der Mehltauentwicklung beurteilt.

Das Ergebnis des Versuches zeigt, daß die Verbindung 30 bei Anwendung als 0,005 %ige Spritzbrühe eine sehr gute fungizide Wirkung hat (beispielsweise 100 %).

**Ansprüche** (für die Vertragsstaaten : BE, CH, DE, FR, IT, LI, NL, SE)

1. Pyridincarbinol der Formel

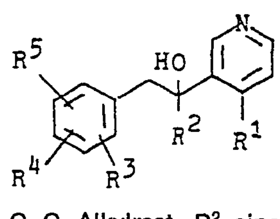

in der $R^1$ Wasserstoff oder einen $C_1$-$C_6$-Alkylrest, $R^2$ einen $C_1$-$C_6$-Alkylrest, $R^3$ und $R^4$ Chlor und $R^5$ Wasserstoff bedeuten und die pflanzenphysiologisch verträglichen Säureadditionssalze dieser Verbindung.

2. Fungizid, enthaltend ein Pyridincarbinol gemäß Anspruch 1.

3. Fungizid, enthaltend einen inerten Trägerstoff und ein Pyridincarbinol gemäß Anspruch 1.

4. Verfahren zur Herstellung eines Fungizids, dadurch gekennzeichnet, daß man einen inerten Trägerstoff vermischt mit einem Pyridincarbinol gemäß Anspruch 1.

5. Verfahren zur Bekämpfung von Pilzen, dadurch gekennzeichnet, daß man die Pilze oder die vor Pilzbefall zu schützenden Pflanzen, Flächen oder Saatgüter behandelt mit einer fungiziden Menge eines Pyridincarbinols gemäß Anspruch 1.

6. Verfahren zur Herstellung eines Pyridincarbinols gemäß Anspruch 1, dadurch gekennzeichnet, daß man ein Acylpyridin der Formel

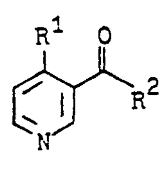

in der $R^1$ und $R^2$ die im Anspruch 1 genannten Bedeutungen haben in Gegenwart eines Lösungsmitteis umsetzt mit einer metallorganischen Verbindung der Formel

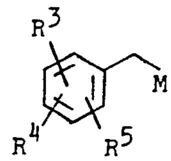

in der M = Li, Na, MgCl oder MgBr bedeutet und $R^3$, $R^4$ und $R^5$ die im Anspruch 1 genannten Bedeutungen haben.

7. Pyridincarbinol gemäß Anspruch 1 der Formel

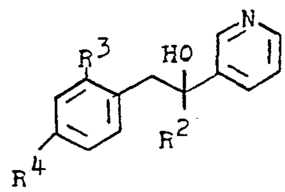

in der $R^2$ $C_1$-$C_6$-Alkyl, $R^3$ Chlor und $R^4$ Chlor bedeuten und die Hydrochloride dieser Verbindung.

8. Fungizid, enthaltend ein Pyridincarbinol gemäß Anspruch 1 der Formel

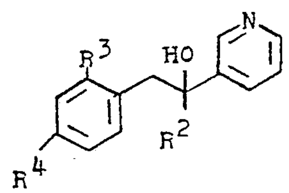

in der $R^2$ $C_1$- bis $C_6$-Alkyl, $R^3$ Wasserstoff oder Chlor und $R^4$ Chlor bedeuten und die Hydrochloride dieser Verbindung.

**Ansprüche** (für den Vertragsstaat AT)

1. Fungizid, enthaltend ein Pyridincarbinol der Formel

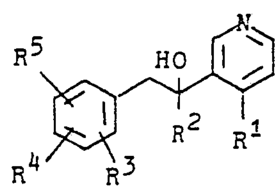

in der $R^1$ Wasserstoff oder einen $C_1$-$C_6$-Alkylrest, $R^2$ einen $C_1$-$C_6$-Alkylrest, $R^3$ und $R^4$ Chlor und $R^5$ Wasserstoff bedeuten und die Pflanzenphysiologisch verträglichen Säureadditionssalze dieser Verbindung.

2. Fungizid, enthaltend einen inerten Trägerstoff und ein Pyridincarbinol gemäß Anspruch 1.

3. Verfahren zur Herstellung eines Fungizids, dadurch gekennzeichnet, daß man einen inerten Trägerstoff vermischt mit einem Pyridincarbinol gemäß Anspruch 1.

4. Verfahren zur Bekämpfung von Pilzen, dadurch gekennzeichnet, daß man die Pilze oder die vor Pilzbefall zu schützenden Pflanzen, Flächen oder Saatgüter behandelt mit einer fungiziden Menge eines Pyridincarbinols gemäß Anspruch 1.

5. Verfahren zur Herstellung eines Pyridincarbinols gemäß Anspruch 1, dadurch gekennzeichnet, daß man ein Acylpyridin der Formel

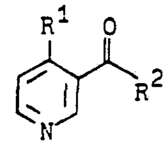

in der $R^1$ und $R^2$ die im Anspruch 1 genannten Bedeutungen haben in Gegenwart eines Lösungsmittels umsetzt mit einer metallorganischen Verbindung der Formel

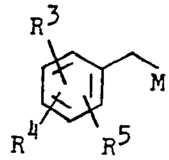

in der M = Li, Na, MgCl oder MgBr bedeutet und $R^3$, $R^4$ und $R^5$ die im Anspruch 1 genannten Bedeutungen haben.

6. Fungizid, enthaltend ein Pyridincarbinol gemäß Anspruch 1 der Formel

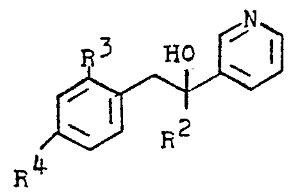

in der $R^2$ $C_1$- bis $C_6$-Alkyl, $R^3$ Chlor und $R^4$ Chlor bedeuten und die Hydrochloride dieser Verbindung.

**Claims** (for the Contracting States : BE, CH, DE, FR, IT, LI, NL, SE)

1. A pyridinecarbinol of the formula

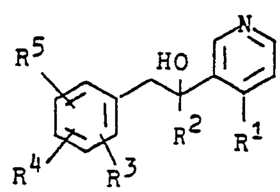

where $R^1$ is hydrogen or $C_1$-$C_6$-alkyl, $R^2$ is $C_1$-$C_6$-alkyl and $R^3$ and $R^4$ are each chlorine, and $R^5$ is hydrogen, and the plant-physiologically tolerated acid addition salts of this compound.

2. A fungicide containing a pyridinecarbinol as claimed in claim 1.

3. A fungicide containing an inert carrier and a pyridinecarbinol as claimed in claim 1.

4. A process for producing a fungicide, wherein an inert carrier is mixed with a pyridinecarbinol as claimed in claim 1.

5. A process for combating fungi, wherein the fungi or the plants, areas or seed to be protected against fungus attack are treated with a fungicidal amount of a pyridinecarbinol as claimed in claim 1.

6. A process for manufacturing a pyridinecarbinol as claimed in claim 1, wherein an acylpyridine of the formula

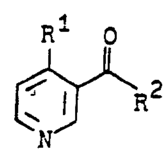

where $R^1$ and $R^2$ have the meanings given in claim 1, is reacted, in the presence of a solvent, with an organometallic compound of the formula

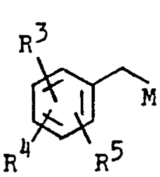

where M is Li, Na, MgCl or MgBr and $R^3$, $R^4$ and $R^5$ have the meanings given in claim 1.

7. A pyridinecarbinol as claimed in claim 1, of the formula

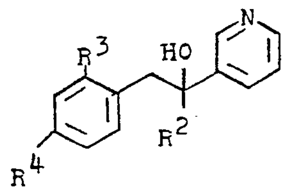

where $R^2$ is $C_1$-$C_6$-alkyl, $R^3$ is chlorine and $R^4$ is chlorine, and the hydrochlorides of this compound.

8. A fungicide containing a pyridinecarbinol as claimed in claim 1, of the formula

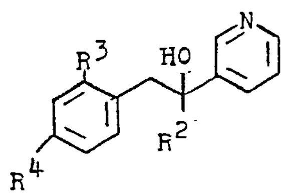

where $R^2$ is $C_1$-$C_6$-alkyl, $R^3$ is hydrogen or chlorine and $R^4$ is chlorine, or a hydrochloride of this compound.

**Claims** (for the Contracting State AT)

1. A fungicide containing a pyridinecarbinol of the formula

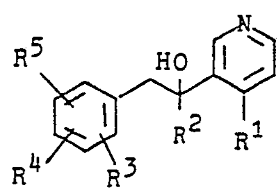

where $R^1$ is hydrogen or $C_1$-$C_6$-alkyl, $R^2$ is $C_1$-$C_6$-alkyl and $R^3$ and $R^4$ are each chlorine and $R^5$ is hydrogen, or a plant-physiologically tolerated acid addition salt of this compound.

2. A fungicide containing an inert carrier and a pyridinecarbinol as defined in claim 1.

3. A process for producing a fungicide, wherein an inert carrier is mixed with a pyridinecarbinol as defined in claim 1.

4. A process for combating fungi, wherein the fungi or the plants, areas or seed to be protected against fungus attack are treated with a fungicidal amount of a pyridinecarbinol as defined in claim 1.

5. A process for manufacturing a pyridinecarbinol as defined in claim 1, wherein an acylpyridine of the formula

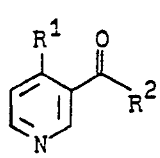

where $R^1$ and $R^2$ have the meanings given in claim 1, is reacted, in the presence of a solvent, with an organometallic compound of the formula

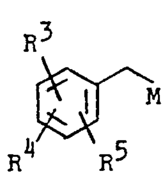

where M is Li, Na, MgCl or MgBr and $R^3$, $R^4$ and $R^5$ have the meanings given in claim 1.

6. A fungicide containing a pyridinecarbinol as defined in claim 1, of the formula

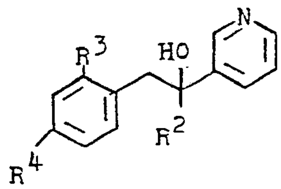

where $R^2$ is $C_1$-$C_6$-alkyl, $R^3$ is hydrogen or chlorine and $R^4$ is chlorine, or a hydrochloride of this compound.

**Revendications** (pour les Etats contractants : BE, CH, DE, FR, IT, LI, NL, SE)

1. Pyridincarbinol de formule

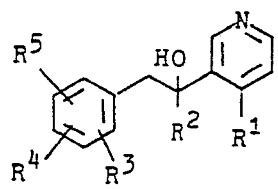

dans laquelle $R^1$ représente l'hydrogène ou un reste alkyle en $C_1$-$C_6$, $R^2$ un reste alkyle en $C_1$-$C_6$, $R^3$ et $R^4$ le chlore et $R^5$ l'hydrogène, et les sels d'addition d'acide de ce composé, compatibles, du point de vue physiologie des plantes.

2. Fongicide, contenant un pyridincarbinol selon la revendication 1.

3. Fongicide, contenant un support inerte et un pyridincarbinol selon la revendication 1.

4. Procédé de préparation d'un fongicide, caractérisé par le fait que l'on mélange un support inerte avec un pyridincarbinol selon la revendication 1.

5. Procédé de lutte contre des champignons, caractérisé par le fait que l'on traite les champignons ou les plantes, surfaces ou semences, à protéger contre l'attaque de champignons, avec une quantité fongicide d'un pyridincarbinol selon la revendication 1.

6. Procédé de préparation d'un pyridincarbinol selon la revendication 1, caractérisé par le fait que l'on fait réagir, en présence d'un solvant, une acylpyridine de formule

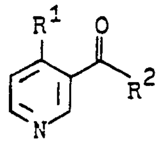

dans laquelle $R^1$ et $R^2$ ont les significations indiquées dans la revendication 1, avec un composé organométallique de formule

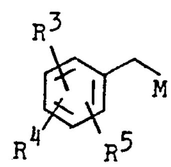

dans laquelle M = Li, Na, MgCl ou MgBr et $R^3$, $R^4$ et $R^5$ ont les significations indiquées dans la revendication 1.

7. Pyridincarbinol selon la revendication 1, de formule

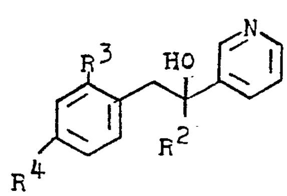

dans laquelle $R^2$ représente un alkyle en $C_1$-$C_6$, $R^3$ du chlore et $R^4$ du chlore, et le chlorhydrate de ce composé.

8. Fongicide, contenant un pyridincarbinol selon la revendication 1, de formule

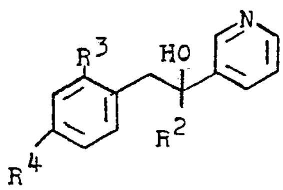

dans laquelle $R^2$ représente un alkyle en $C_1$ à $C_6$, $R^3$ l'hydrogène ou le chlore et $R^4$ le chlore, et le chlorhydrate de ce composé.

**Revendications** (pour l'Etat contractant AT)

1. Fongicide contenant un pyridincarbinol de formule

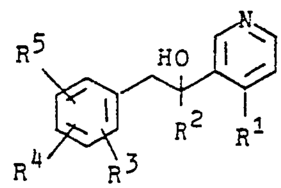

dans laquelle $R^1$ représente l'hydrogène ou un reste alkyle en $C_1$-$C_6$, $R^2$ un reste alkyle en $C_1$-$C_6$, $R^3$ et $R^4$ le chlore et $R^5$ l'hydrogène, et les sels d'addition d'acide de ce composé, compatibles, du point de vue physiologie des plantes.

2. Fongicide, contenant un support inerte et un pyridincarbinol selon la revendication1.

3. Procédé de préparation d'un fongicide, caractérisé par le fait que l'on mélange un support inerte avec un pyridincarbinol selon la revendication 1.

4. Procédé de lutte contre les champignons, caractérisé par le fait que l'on traite les champignons ou les plantes, surfaces ou semences, à protéger contre l'attaque de champignons, avec une quantité fongicide d'un pyridincarbinol selon la revendication 1.

5. Procédé de préparation d'un pyridincarbinol selon la revendication 1, caractérisé par le fait que l'on fait réagir, en présence d'un solvant, une acylpyridine de formule

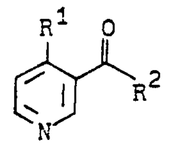

dans laquelle $R^1$ et $R^2$ ont les significations indiquées dans la revendication 1, avec un composé organométallique de formule

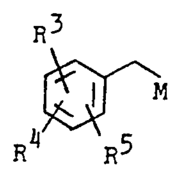

dans laquelle M = Li, Na, MgCl ou MgBr et $R^3$, $R^4$ et $R^5$ ont les significations indiquées dans la revendication 1.

6. Pyridincarbinol selon la revendication 1, de formule

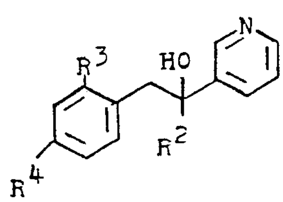

dans laquelle $R^2$ représente un alkyle en $C_1$-$C_6$, $R^3$ du chlore et $R^4$ du chlore, et le chlorhydrate de ce composé.